# EUROPEAN PATENT APPLICATION

(11) **EP 4 112 597 A1**
(43) Date of publication of application: **04.01.2023**
(21) Application number: 21759585.9
(22) Date of filing: 26.02.2021
(51) Int. Cl.: C07C 233/47, C07C 237/22, C07C 323/52, G01N 31/00

(54) **STABILIZATION OF POLYSULFIDE**

(30) Priority: 28.02.2020 JP 2020034471
(71) Applicant: University Public Corporation Osaka, Osaka-shi, Osaka 545-0051 (JP); Bio-Xcelerator, Inc., Tokyo 160-0016 (JP)
(72) Inventor: IHARA, Hideshi, Sakai-shi, Osaka 599-8531 (JP); KASAMATSU, Shingo, Sakai-shi, Osaka 599-8531 (JP)
(74) Representative: Mewburn Ellis LLP
(86) International application number: PCT/JP2021/007397
(87) International publication number: WO 2021/172523

(57) **Abstract**

The present invention is a new tyrosine derivative described below to inhibit hydrolysis of polysulfide.

The new tyrosine derivative formed according to a chemical formula indicated below, wherein one of a-c is "OH" and the rest are "H";
d is CₙH₂ₙα, where α is "H" or a "halogen atom," the halogen atom is preferably "I," and
n is an integer from 1 to 5; and
e is (NH)ₓ(CO)_{y}C_{z}H_{2z}β, where x is 1 or 0, y is 1 or 0, z is an integer from 1 to 5, β is "H" or a "halogen atom,".

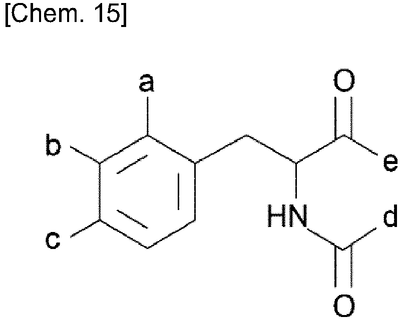

## Description

### TECHNICAL FIELD

The present invention relates to a new tyrosine derivative(s). Particularly, the invention relates to materials, compounds, compositions, reagents, and so on which contain a tyrosine derivative(s) as their main components and are suited to stabilize active sulfur species so that they can be detected.

### BACKGROUND ART

The physiological importance of reactive sulfur species / active sulfur species (RSS) such as cysteine hydropolysulfide (CysSSH) and glutathione polysulfide (GSSH) is described in, for example, NATURE COMMUNICATIONS 8: 1177, Cysteinyl-tRNA synthetase governs cysteine polysulfidation and mitochondrial bioenergetics, 27 October 2017.

Cysteine hydropolysulfide (CysSSH) occurs in abundant quantities in various living organisms, but little is known about its bio synthesis and physiologic functions. Extensive persulfide formation is apparent in cysteine-containing proteins in *Escherichia coli* and mammalian cells and is believed to result from post-translational processes including hydrogen-sulfide-related chemical reactions.

Effective CysSSH synthesis from a substrate L-cysteine exists, which is a reaction catalyzed by prokaryotic and mammalian cysteinyl-tRNA synthetases (CARSs). Targeted disruption of genes which encode mitochondrial CARSs in mice and human cells shows that CARSs have a crucial role in endogenous CysSSH production and suggests that these enzymes serve as the principal cysteine persulfide synthases in vivo. CARSs also catalyze co-translational cysteine polysulfidation and are involved in the regulation of mitochondrial biosynthesis and bioenergetics. Therefore, investigating CARS-dependent persulfide production may possibly clarify abnormal redox signal transduction in physiological and pathophysiological conditions and suggest therapeutic targets based on oxidative stress and mitochondrial dysfunction.

In vivo kinetics of a considerable amount of RSS generated endogenously and ubiquitously in both prokaryotes and eucaryotes will be clarified by establishing active sulfur metabolomics analysis by using RSS metabolic profiling. A quantitative analysis method for the active sulfur species from biological samples (such as mice tissues and cultured cells) was reported in 2014 (Ida T. et al., PNAS 2014).

Also, as a system for detecting protein polysulfide (active sulfur molecules in protein cysteine residue), there were reported, for example, a modified tag switch assay (Ida T. et al., PNAS 2014; Mustafa A. Science 2009), a maleimide assay (Gao XH. et al., eLife 2016, Yang R. et al., Immunity 2015), a pulldown assay (Doka E. Et al., Sci. Adv. 2016), and a biotin-polyethylene glycol-conjugated maleimide gel shift assay (Jung M. et al., BBRC 2016).

The in vivo kinetics of a considerable amount of RSS generated endogenously and ubiquitously in both prokaryotes and eucaryotes will be clarified by establishing the active sulfur metabolomics analysis by using the RSS metabolic profiling.

Chemical properties of polysulfides are not completely understood or elucidated due to their reactivity or complicated redox active properties. It is known that anyone of compounds which are publicly known as detecting reagents strongly promote decomposition of the active sulfur species. Redox Biology Volume 21 (2019) 101096 Science Direct clarified a system using β-(4-hydroxyphenyl) ethyl iodoacetamide (HPE-IAM) as an improved version of the quantitative analysis method for the active sulfur species from the biological samples.

### CITATION LIST

### NON-PATENT LITERATURE

NPL 1: Redox Biology Volume 21 (2019) 101096 Science Direct

### SUMMARY OF THE INVENTION

### PROBLEMS TO BE SOLVED BY THE INVENTION

The inventor established the active sulfur metabolomics analysis by using the RSS metabolic profiling. Accordingly, this has clarified the in vivo kinetics of the considerable amount of RSS generated endogenously and ubiquitously in both prokaryotes and eucaryotes.

However, the inventor of the present invention has found through examination that even when the HPE-IAM is used, the decomposition of the RSS (active sulfur species) occurs.

So, it is an object of this invention to realize the stabilization of the RSS in order to make it possible to analyze the RSS.

### MEANS TO SOLVE THE PROBLEMS

In order to achieve the above-described object, the present invention provides a new tyrosine derivative. The tyrosine derivative of the present invention is identified by a chemical formula indicated below,
wherein one of a-c is "OH" and the rest are "H";
d is CₙH₂ₙα, where α is "H" or a "halogen atom," the halogen atom is preferably "I," and n is an integer from 1 to 5; and
e is (NH)ₓ(CO)_{y}C_{z}H_{2z}β, where x is 1 or 0, y is 1 or 0, z is an integer from 1 to 5, β is "H" or a "halogen atom,".

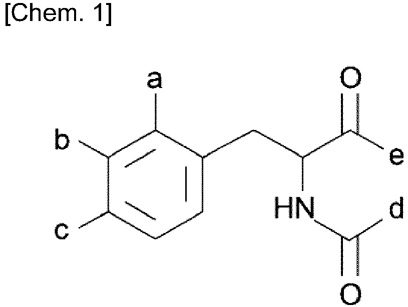

### ADVANTAGEOUS EFFECTS OF THE INVENTION

The stabilization of the RSS can be realized according to the present invention.

### BRIEF DESCRIPTION OF DRAWINGS

Fig. 1 are graphs showing HPLC analysis results of (A) a Tyrosine Methyl Ester sample and (B) a reaction solution;
Fig. 2 is a graph showing HPLC detection results of a partially purified liquid;
Fig. 3 are graphs showing analysis results of the partially purified liquid with LC-MC/MS;
Fig. 4 are graphs showing analysis results of the partially purified liquid with the LC-MC/MS;
Fig. 5 are graphs of reactivity test results of tyrosine derivatives and cysteine;
Fig. 6 is a graph showing the results of a stabilization test for active sulfur molecules of the respective alkylating agents containing a new tyrosine derivative;
Fig. 7 is a graph showing the results of a pH influence test for a GS-SS-SG protection effect of TME-IAM;
Fig. 8 is a graph showing the results of a reaction mole ratio influence test for the GS-SS-SG protection effect of TME-IAM;
Fig. 9 are graphs showing the results of a reactivity test of TME-IAM with respect to, for example, GS-SS-SG;
Fig. 10 is a diagram showing a reactivity comparison of TME-IAM and HPE-IAM with respect to cysteine hydropolysulfide [CysS-(S)ₙ-H];
Fig. 11 are graphs showing cell permeability of TME-IAM;
Fig. 12 are graphs showing reactivity of TME-IAM with respect to hydrogen sulfide; and
Fig. 13 is a graph showing the GS-SS-SG protection effect of N-iodoacetyl tyrosine.

### DESCRIPTION OF EMBODIMENTS

The inventor of the present invention elucidated that a tyrosine derivative(s) according to the present invention has the stabilization effect for various polysulfides. For example, the protection effect for the decomposition of glutathione polysulfides (oxidized glutathione trisulfide and oxidized glutathione tetrasulfide) is caused by hydroxyphenyl residues of the tyrosine derivative with respect to alkali hydrolysis of the polysulfides.

The hydrolysis of polysulfide is triggered by hydroxyl anions which act on the polysulfide, causes heterolytic cleavage to the polysulfide, and decomposes the polysulfide to thiolate and sulfenic acid. The hydrolysis is promoted by an alkylating agent (such as IAM) and dimedone.

The tyrosine derivative prevents electrophilic decomposition caused by alkaline pH. When the stabilization of polysulfide which is induced by a hydroxyphenyl part of the tyrosine derivative remarkably improves the understanding of the chemical properties of polysulfide and can be applied well to all kinds of biological samples including clinical specimens, it is useful for the RSS metabolomics analysis.

The tyrosine derivative of the present invention is identified by the following chemical formula.

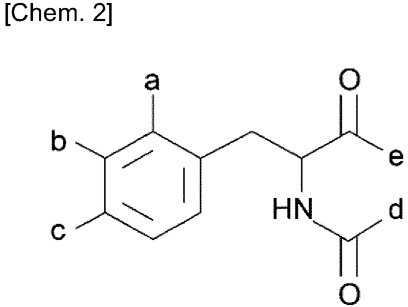

One of a-c is "OH" and the rest are "H";
d is CₙH₂ₙα, where α is "H" or a "halogen atom," the halogen atom is preferably "I," and n is an integer from 1 to 5; and
e is (NH)ₓ(CO)_{y}C_{z}H_{2z}β, where x is 1 or 0, y is 1 or 0, z is an integer from 1 to 5, β is "H" or a "halogen atom,".

The tyrosine derivative(s) includes the following compounds.

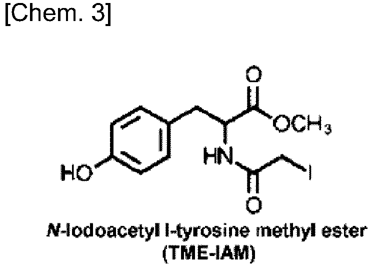

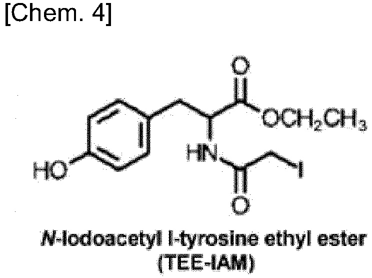

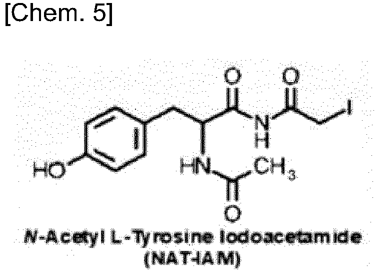

The inventor of the present invention has confirmed through PubChem (https://pubchem.ncbi.nlm.nih.gov/) that these tyrosine derivatives are new substances. The tyrosine derivatives of the present invention are also iodoacetamide derivatives.

The reaction formulas indicated below show the outline of a chemical reaction which explains that polysulfide undergoes hydrolysis and is decomposed, and which is a reaction between the polysulfide and an electrophilic reagent or dimedone. The decomposition process of GS-SS-SG in the drawing is started by alkali-induced hydrolysis and, as a result, a thiolate and a sulfenyl part are generated.

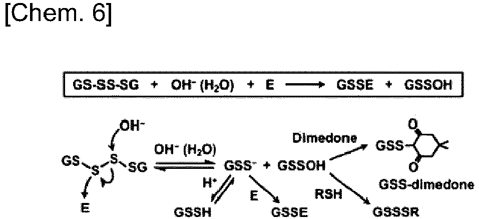

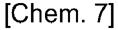

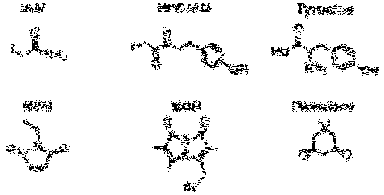

If regarding a specified sulfur residue, for example, "-SS-" of GS-SS-SG, its neighboring sulfur is hydroxylated (OH-added) and GSS-OH is excluded, cleavage is conducted by an electrophile and a GSS-E adduct is generated by electrophilic alkylation, thereby finally causing electrophilic decomposition of polysulfide involving hydrolysis. Attention should be paid to the fact that even alkylating agents containing electrophilic IAM, such as TME-IAM, TEE-IAM, and NAT-IAM, have a strong polysulfide stabilization effect as long as their hydroxyl part such as TME is functionally active to inhibit alkali hydrolysis.

Regarding the alkylating agent such as TME-IAM, halogen (iodine) acts on -SH (reduced S) and forms -S-AM-TME. The hydroxy group of, for example, TME-IAM suppresses hydrolysis of -Sₙ- (n: an integer equal to or more than 2): oxidized sulfur. Another interesting point is that this decomposition does not include a normal redox process which has been made cleared that, for example, it is mediated by thioredoxin and thioredoxin reductase, and which is influenced by a standard endogenous reduction reaction.

The hydroxylation of the polysulfidated residue is antagonized by, for example, TME-IAM and this hydroxylation is completely different from a redox-dependent reaction mediated by a counteractive reduction system, which is endogenously expressed, with ROS.

The thiolate part is rapidly alkylated by a strong electrophilic reagent such as monobromobimane (MBB) or N-ethyl maleimide (NEM). The sulfenyl part reacts with other thiol compounds, or dimedone of a sulfenic acid probe. Therefore, the electrophilic reagent and the dimedone cause a shift in equilibrium of the alkali hydrolysis of the polysulfide and promote the decomposition of the polysulfide.

It is well known that the RSS is generated abundantly endogenously in many species. A new family of cysteine persulfur synthetic enzymes which mainly have polyfunctional property, that is, cysteinyl-tRNA synthetase (CARS) was discovered not only in a translation process during biosynthesis of protein, but as a specific enzyme for generating persulfide or polysulfide from cysteine which is a substrate. What is very important in relation to this discovery is that a mitochondria isoform of CARS (CARS2) is the major enzyme which is involved in most of active sulfur species formed inside a living body, and contributes to bioenergy of mitochondria, that is, sulfur breathing.

In order to investigate these sulfide metabolic pathways, a reactive sulfur metabolomics analysis was firstly developed. This was done by using a liquid chromatography mass spectrometry (LC-MS) and combining with trapping or derivatization of the active sulfur species by a tyrosine derivative. The specificity of this approach depends on selectivity of chemical reactions of the tyrosine derivative with various RSS and polysulfides. Therefore, it is effective to use a compound(s) having an OH group such as tyrosine derivatives.

Consequently, the electrophilic decomposition of the polysulfide can be minimized and it becomes possible to implement specific nucleophilic substitution of sulfhydryl of various hydropolysulfides. As a result, specific detection and quantification of RSS are guaranteed through the sulfur metabolomics analysis.

However, as indicated with the aforementioned reaction formula, harsh electrophiles such as MBB and NEM may possibly be involved in a nucleophilic attack to the sulfur residue of the polysulfide, and this may lead to a wide range of polysulfide decomposition. Practically, this process is started by alkali-induced hydrolysis of the polysulfide and, as a result, a thiolate residue is generated from the polysulfide and is easily alkylated by MBB and NEM; however, this was not recognized with the tyrosine derivatives.

Furthermore, this polysulfide decomposition, which is caused by the shift of the alkali hydrolysis equilibrium when the dimedone reacts with sulfenyl of a thiol or polythiol part, is promoted by the dimedone. Therefore, unless the unique sulfide chemistry is correctly understood, the specific metabolomics analysis cannot be applied to the analysis of the RSS and the reactivity polysulfides contained in abundant quantities in many biological systems.

For example, one of serious problems is that adducts of the harsh reagents such as NEM and MBB and the hydrogen sulfide (H₂S) were frequently observed in a reaction between NEM/MBB and a polysulfide which combined with low molecular weight of cysteine and protein cysteine residue.

There is a high possibility that these adducts may be generated artificially during processing of various samples which use MBB/NEM. Since it is known that CARS can induce polysulfidation of all Cys-containing proteins after the translation or during the translation process, such artificial formation of a bismonosulfide alkyl adduct (R-S-R) seems to be the most serious problem in the metabolomics analysis using the electrophilic compounds, regardless of the reactivity of these electrophilic reagents.

This difficulty exists even in a case where the tyrosine derivatives are used to make it possible to minimize artificial degradation and R-S-R formation. The inventor examined a new mechanistic approach by using tyrosine derivative trapping in combination with LC-tandem mass spectrometry (LC-MS/MS) in order to overcome this technical problem.

In addition to the verification of relatively selective RSS trapping by the tyrosine derivative, the inventor has discovered that the tyrosine derivative itself can stabilize polysulfide. In fact, the inventor has found that when a tyrosine derivative is added to a reaction mixture containing polysulfide, the tyrosine derivative exhibits a strong polysulfide maintaining effect. This effect may possibly be attributable to a chemical structure (a hydroxyphenyl group or an iodoacetamide group) which is similar among a plurality of tyrosine derivatives.

The polysulfide stabilization effect of the tyrosine derivatives and the hydroxyphenylcontaining compound have the significant meanings for better understanding of a molecular mechanism to maintain the formation of a wide range of protein polysulfides in many proteins.

The polysulfides have a reduced form and an oxidized form and R-(S)ₙ-SH (n=1,2,....) is the reduced form and R-(S)ₙ-R (n=2,3,....) is the oxidized form. The reduced-form polysulfide (thiol compound) may preferably be the aforementioned hydroxy compound, for the sake of adding hydrophobicity to enable the LC-MSMS analysis and stabilizing the polysulfide, so that such hydroxy compound can combine with the thiol group of the reduced-form polysulfide and be stabilized. This compound may be the aforementioned TME-IAM as indicated below.

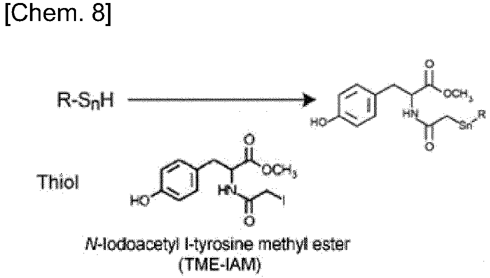

The addition of hydrophobicity to the polysulfide makes reverse-phase HPLC separation possible. By labeling the tyrosine derivative, it can be used as an internal standard substance. "I" in the tyrosine derivative may be substituted with another halogen such as Cl, F, or Br. The OH bonded to the benzene ring may be bonded at an ortho or meta position. The benzene ring may be substituted with aliphatic chain hydrocarbon or aliphatic cyclic hydrocarbon. The benzene may be substituted with a polycyclic aromatic compound such as naphthalene or anthracene. Also, the benzene may be substituted with a heterocyclic compound.

The following are examples of the reduced sulfides and polysulfides.

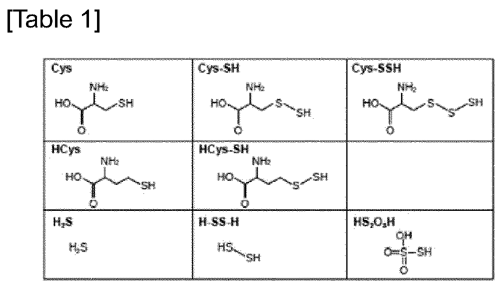

Furthermore, the following are examples of the oxidized-form polysulfides.

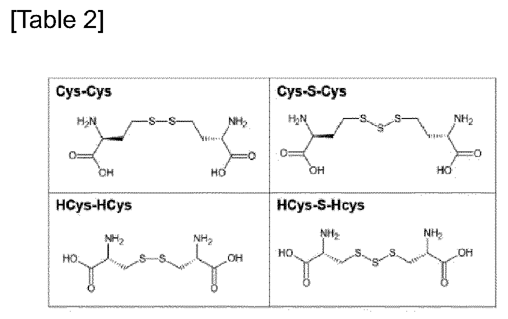

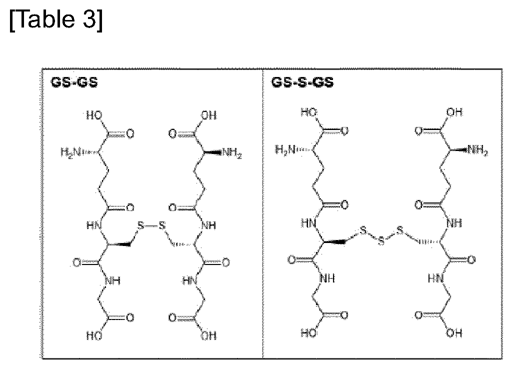

Next, the synthesis and qualitative analysis of the tyrosine derivatives will be explained. 1-1 Testing Materials and Methods

1-1-1 Dehydration Condensation Reaction between Tyrosine Methyl Ester and Iodoacetic Acid (TME-IAM Synthesis)

### 1-1-1-1 Synthesis

One mmol of L-Tyrosine Methyl Ester was dissolved in *N*,*N*-dimethylformamide, to which 2.2 mmol of *N*,*N*'-dicyclohexyl carbodiimide as a dehydration condensation agent and 1 mmol of iodoacetic acid were added, and the obtained solution was stirred on ice for 2 hours and then stirred at room temperature for one hour.

### Reagents

*N*-iodoacetyl tyrosine methyl ester (TME-IAM) synthesis
Tyrosine Methyl Ester (Tokyo Chemical Industry Co., Ltd.) 195.2 mg
*N*,*N* -dimethylformamide (KATAYAMA CHEMICAL INDUSTRIES Co., Ltd.) 2 mL *N*,*N*'-dicyclohexyl carbodiimide (nacalai tesque) 453.8 mg
Iodoacetic acid (nacalai tesque) 186.0 mg

### 1-1-1-2 Checking of Product

The Tyrosine Methyl Ester solution and a reaction solution were diluted to check the synthesized TME-IAM, and then the absorbance at 220 nm, 250 nm, and 275 nm was analyzed by using high-speed liquid chromatography (high performance liquid chromatography, HPLC) and a reaction product was checked.

### Reagents

0.1% Formic acid
Formic Acid (abt. 99%) (FUJIFILM) 1 mL
Ultrapure water 999 mL
Methanol for LC/MS (KANTO CHEMICAL CO., INC.)

### Equipment

Pump: PU-2085 plus (JASCO)
Autosampler: AS-2051 plus (JASCO)
Detector: MD-2010 plus (JASCO)
Degasser: DG-2085-54 (JASCO)
Gradient mixer: MX-2080-32 (JASCO)
Column oven: CO-2065 plus (JASCO)

HPLC Conditions
A buffer: 0.1% formic acid
B buffer: methanol

### Gradient:

**[Table 4]**

| Time (min) | 0 | 14 | 18 | 18.1 | 22 |
|---|---|---|---|---|---|
| A buffer (%) | 100 | 0 | 0 | 100 | 100 |
| B buffer (%) | 0 | 100 | 100 | 0 | 0 |

Flow speed: 1 mL/min
Inflow: 5 µL
Column: Mightysil RP-18 GP 75-3.0 (5 µm) (KANTO CHEMICAL CO., INC.)

### 1-1-2 Purification of TME-IAM

### 1-1-2-1 Partial Purification with Wakogel (C18)

After adding 4 mL of methanol and 1 g of Wakogel (C18) to the reaction solution, 6 mL of 0.1% formic acid was added to the obtained solution while stirring it. An empty reservoir was filled this solution, which was then filtered. Furthermore, 1 mL of methanol and 1 mL of 0.1% formic acid were mixed and then added to the empty reservoir and an eluate was recovered. This eluate was recognized as a partially purified liquid.

### Reagents

0.1% Formic acid
Methanol (KANTO CHEMICAL CO., INC.)
Wakogel R100C18 (Wako)

### 1-1-2-2 Checking of Partial Purification

After diluting the partially purified liquid to check the purification of TME-IAM, the absorbance was analyzed at 220 nm, 250 nm, and 275 nm under similar conditions to those of 1-1-1-2 and the recovery of the reaction product was checked.

### 1-1-2-3 Checking of Product in Partially Purified Liquid

After diluting the partially purified liquid to check the existence of TME-IAM in the purified liquid, the existence of TME-IAM was checked by a mass spectrometry method by using an HPLC-tandem-type mass spectrometer (LC-MS/MS).

### Reagents

0.1% Formic acid
Methanol for LC/MS (KANTO CHEMICAL CO., INC.)

### Equipment

Mass spectrometer: Xevo TQD (Waters)
HPLC: Alliance HPLC e2695 (Waters)

HPLC Conditions
A buffer: 0.1% formic acid
B buffer: methanol (for LC/MS)

### Gradient:

**[Table 5]**

| Time (min) | 0 | 5 | 6.5 | 6.6 | 12 |
|---|---|---|---|---|---|
| A buffer (%) | 99 | 1 | 1 | 99 | 99 |
| B buffer (%) | 1 | 99 | 99 | 1 | 1 |

Flow speed: 0.3 mL/min
Inflow: 5 µL
Column: Mightysil RP-18 GP 50-2.0 (5 µm) (KANTO CHEMICAL CO., INC.)

### MS Scan Conditions

ES+
Q1 scan range(*m*/*z*): 50-600
Time (min): 0-9
Cone voltage (V): 10-35

### 1-1-2-4 Purification of TME-IAM

The partially purified liquid was injected into HPLC using a fractionation column and fractionation for every 2 minutes was recovered. The isolation of TME-IAM was confirmed by analyzing the absorbance of the obtained fractionations at 220 nm, 250 nm, and 275 nm under similar conditions to those of 1-1-1-2.

### Reagents

0.1% Formic acid
Methanol (KANTO CHEMICAL CO., INC.)

### Equipment

Pump: PU-2089 plus (JASCO)
Detector: 875-UV (JASCO)

HPLC Fractionating Conditions
A buffer: 0.1% Formic acid
B buffer: methanol

### Gradient:

**[Table 6]**

| Time (min) | 0 | 200 | 240 |
|---|---|---|---|
| A buffer (%) | 100 | 0 | 0 |
| B buffer (%) | 0 | 100 | 100 |

Flow speed: 3 mL/min
Detected wavelength: 275 nm
Inflow: 1 mL
Column: CAPCELL PAK C18 UG80 5 µm (SHISEIDO)

### 1-2 Results

### 1-2-1 Checking of Product

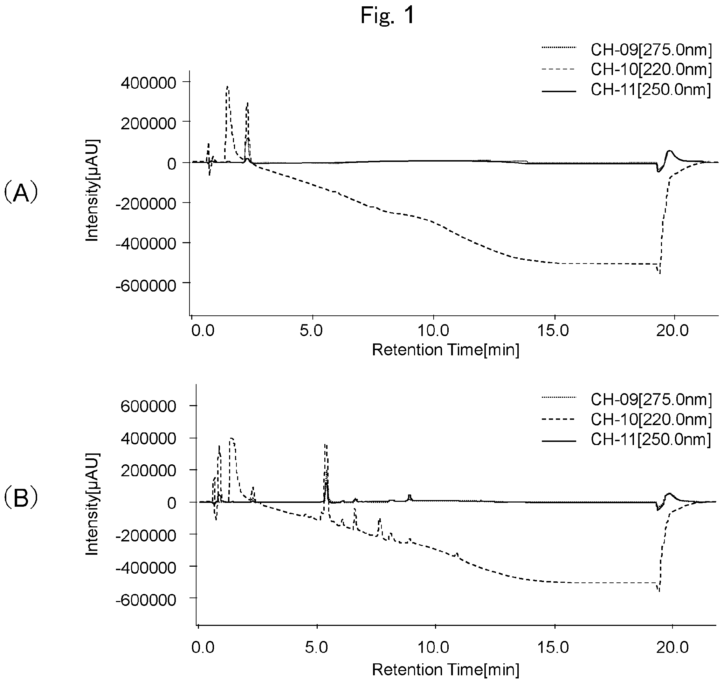

A new peak different from that of the Tyrosine Methyl Ester sample (Fig. 1(A)) was detected in the reaction solution (Fig. 1(B)) by the HPLC analysis.

### 1-2-2 Purification of TME-IAM

### 1-2-2-1 Checking of Partially Purified Fractions

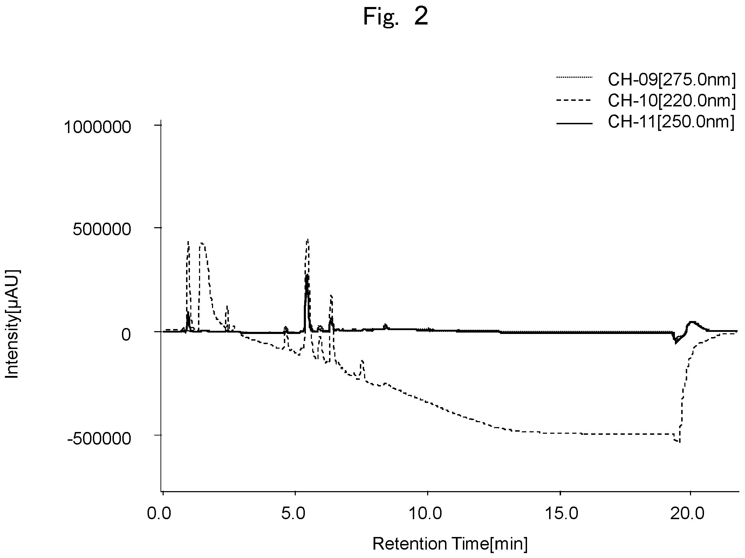

It was confirmed by the HPLC analysis that the reaction product was recovered in the partially purified fractions (Fig. 2).

### 1-2-2-2 Checking of Product in Partially Purified Liquid

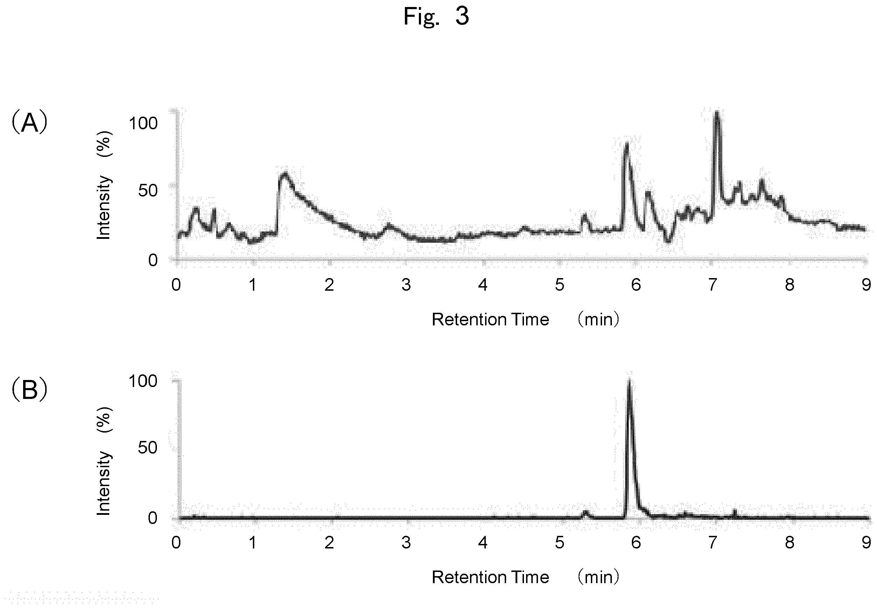

The results of analyzing the partially purified liquid by the LC-MC/MS are shown in Fig. 3(A). When a chromatogram of *m*/*z* = 364 was extracted from the obtained chromatogram, a peak which seemed to be that of TME-IAM was observed (Fig. 3(B)).

### 1-2-2-3 Purification of Partially Purified Liquid

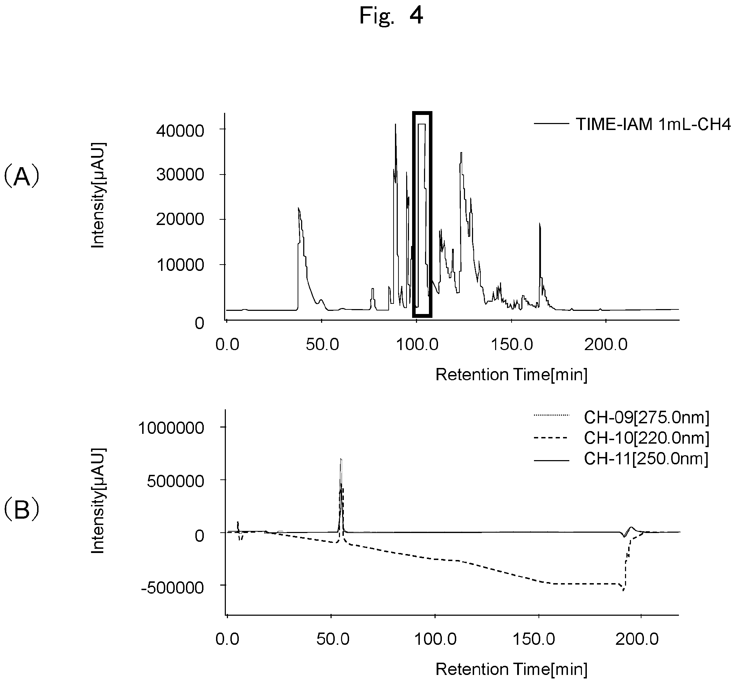

The results of purifying the partially purified fractions with HPLC using the distribution column are shown in Fig. 4(A). After performing the HPLC analysis of each fraction, only the fraction(s) containing TME-IAM was recovered and recognized as a purification sample of TME-IAM (Fig. 4(B)).

The above-described synthesis and results are regarding TME-IAM. Regarding TEE-IAM, it is only necessary to change the starting material for TME-IAM from L-Tyrosine Methyl Ester (TME) to L-Tyrosine Ethyl Ester (TEE); and regarding NAT-IAM, it is only necessary to change the starting material to *N*-Acetyl-L-tyrosine (NAT). Regarding the qualitative analysis method and the judgment of the results, the same method and judgment as those applied to TME-IAM may be used.

Next, the reactivity between the tyrosine derivative according to the present invention and the active sulfur species was examined.

The following were mixed: 50 µL of 200 mM phosphate buffer (pH 7.5) (final concentration: 50 mM), 2 µL of 100 mM alkylating agent (TME-IAM, TEE-IAM, NAT-IAM, HPE-IAM) or DMSO (final concentration: 1 mM), 2 µL of 10 mM cysteine (final concentration: 0.1 mM), and 146 µL of ultrapure water; and the mixture was incubated at 37°C while shielding light. One, 3, and 8 hours after the start of the reaction, 10 µL of the reaction solution and 90 µL of 0.1% formic acid were mixed, thereby terminating the reaction. By performing the LC-MS/MS by using unreacted 10 µM cysteine as standard, a residual amount of the cysteine in the reaction solution was analyzed and the generation of cysteine adducts by various kinds of alkylating agents was analyzed.

### Equipment

Mass spectrometer: Xevo TQD (Waters)
HPLC: Alliance HPLC e2695 (Waters)

HPLC Gradient Conditions (analysis of residual cysteine amount and generated cystine amount)
A buffer: 0.1% formic-acid-containing acetonitrile
B buffer: 100 mM ammonium formate

**[Table 7]**

| Time (min) | 0 | 5 | 8 | 8.1 | 16 |
|---|---|---|---|---|---|
| A buffer (%) | 99 | 1 | 1 | 99 | 99 |
| B buffer (%) | 1 | 99 | 99 | 1 | 1 |

Flow speed: 0.3 mL/min
Inflow: 5 µL
Column: Intrada amino acid column (50×2.0 mm inner diameter) (Imtakt)

HPLC Gradient Conditions (analysis of cysteine adduct by various kinds of alkylating agents)
A buffer: 0.1% formic acid
B buffer: methanol (for LC/MS)

**[Table 8]**

| Time (min) | 0 | 5 | 7 | 7.1 | 12 |
|---|---|---|---|---|---|
| A buffer (%) | 99 | 1 | **1** | 99 | 99 |
| B buffer (%) | 1 | 99 | 99 | **1** | 1 |

Flow speed: 0.3 mL/min
Inflow: 5 µL
Column: Mightysil RP-18 GP 50-2.0 (5 µm) (KANTO CHEMICAL CO., INC.)

### Multiple Reaction Monitoring Conditions

**[Table 9]**

| Analyte | Precursor ions (*m*/*z*) | Fragment ions (*m*/*z*) | Cone voltage (V) | Collision energy (V) |
|---|---|---|---|---|
| Cysteine | 122.0 | 58.8 | 20 | 20 |
| Cystine | 241.0 | 120.0 | 25 | 15 |
| Cys-AM-HPE | 299.0 | 121.0 | 35 | 30 |
| Cys-AM-TME | 357.1 | 136.0 | 35 | 30 |
| Cys-AM-TEE | 371.4 | 136 | 45 | 25 |
| Cys-AM-NAT | 384.4 | 136 | 30 | 35 |

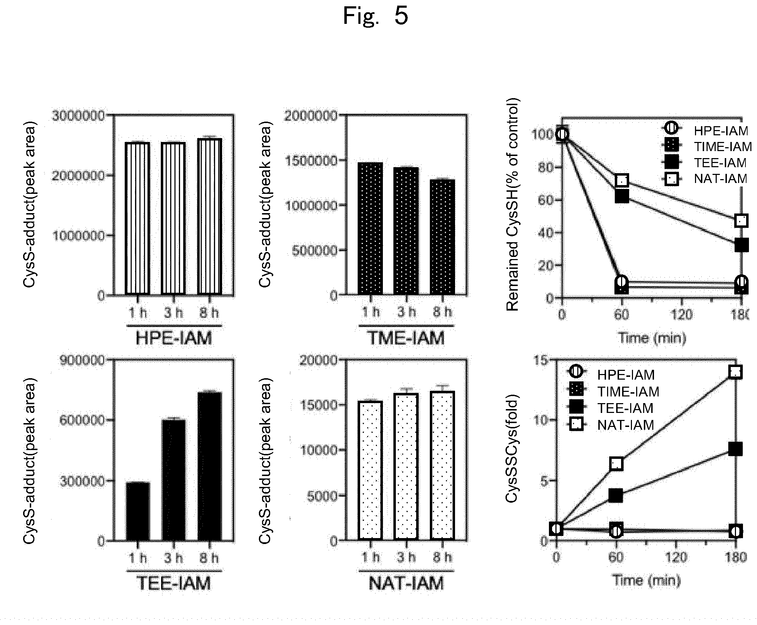

It was confirmed that TME-IAM has the same level of reactivity with the thiol group (cysteine) as that of HPE-IAM having a tyrosine derivative as described in the aforementioned Non-Patent Literature. It was found that TEE-IAM and NAT-IAM have lower reactivity with cysteine than that of TME-IAM and HPE-IAM. The results are shown in Fig. 5. Incidentally, HPE-IAM is expressed as the following chemical formula.

### HPE-IAM:

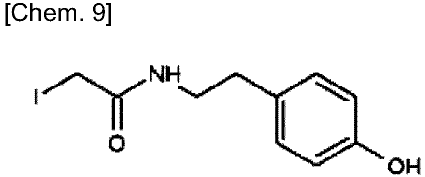

Next, the stabilization effect of active sulfur molecules of each alkylating agent containing the new tyrosine derivative was evaluated. Oxidized glutathione tetrasulfide (GS-SS-SG) was used as a model compound for the active sulfur molecules.

The following were mixed: 50 µL of 200 mM phosphate buffer (pH 7.0) (final concentration: 100 mM), 5 µL of 20 mM alkylating agent (TME-IAM, HPE-IAM, MBB, IAM) or DMSO (final concentration: 1 mM), 10 µL of 100 µM GS-SS-SG (final concentration: 10 µM), and 35 µL of ultrapure water; and the mixture was incubated at 37°C while shielding light. One, 2, 3, and 4 hours after the start of the reaction, 10 µL of the reaction solution and 90 µL of 0.1% formic acid were mixed, thereby terminating the reaction. By performing the LC-MS/MS by using unreacted 1 µM GS-SS-SG as standard, a residual amount of the GS-SS-SG in the reaction solution was analyzed.

### Equipment

Mass spectrometer: Xevo TQD (Waters)
HPLC: Alliance HPLC e2695 (Waters)

### HPLC Gradient Conditions

A buffer: 0.1% formic acid
B buffer: methanol (for LC/MS)

**[Table 10]**

| Time (min) | 0 | 5 | 7 | 7.1 | 12 |
|---|---|---|---|---|---|
| A buffer (%) | 99 | **1** | 1 | 99 | 99 |
| B buffer (%) | 1 | 99 | 99 | **1** | 1 |

Flow speed: 0.3 mL/min
Inflow: 5 µL
Column: Mightysil RP-18 GP 50-2.0 (5 µm) (KANTO CHEMICAL CO., INC.)

### Multiple Reaction Monitoring Conditions

**[Table 11]**

| Analyte | Precursor ions (*m*/*z*) | Fragment ions (*m*/*z*) | Cone voltage (V) | Collision energy (V) |
|---|---|---|---|---|
| GS-SS-SG | 677.1 | 339.0 | 35 | 25 |

It is known that autolysis of GS-SS-SG is caused just incubate at 37°C in neutral pH (pH 7.0); and its decomposition is promoted by adding monobromobimane (MBB) which is a compound with high reactivity among publicly known alkylating agents.

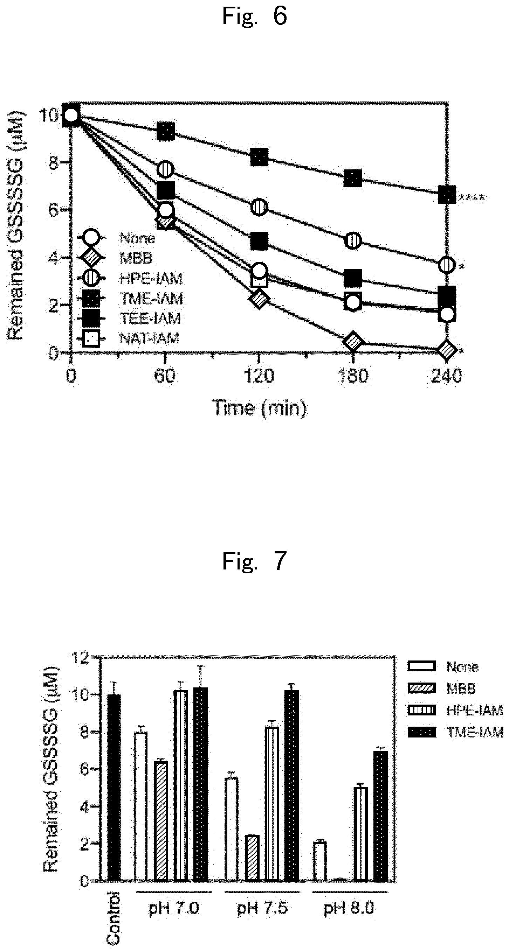

On the other hand, the autolysis of GS-SS-SG was inhibited significantly when adding HPE-IAM, a publicly known iodoacetamide derivative. Furthermore, with a group to which TME-IAM was added, approximately 80% GS-SS-SG remained even after the incubation for 4 hours; and, therefore, it shows that TME-IAM, which is a new alkylating agent, has a high active sulfur molecules stabilization activity. The results are shown in Fig. 6.

Next, the pH influence of the GS-SS-SG protection effect of TME-IAM was evaluated. It has been clarified that the active sulfur molecules GS-SS-SG are stable under acid conditions, while they are very unstable under alkali conditions.

The following were mixed: 50 µL of 200 mM phosphate buffer (pH 7.0, 7.5, 8.0) (final concentration: 100 mM) and 5 µL of 20 mM TME-IAM solution (final concentration: 1 mM), 10 µL of 100 µM GS-SS-SG (final concentration: 10 µM), and 35 µL of ultrapure water; and the mixture was incubated at 37°C while shielding light. One hour after the start of the reaction, 10 µL of the reaction solution and 90 µL of 0.1% formic acid were mixed, thereby terminating the reaction. By performing the LC-MS/MS analysis by using unreacted 1 µM GS-SS-SG as standard, a residual amount of GS-SS-SG in the reaction solution was analyzed.

### Equipment

Mass spectrometer: Xevo TQD (Waters)
HPLC: Alliance HPLC e2695 (Waters)

### HPLC Gradient Conditions

A buffer: 0.1% formic acid
B buffer: methanol (for LC/MS)

**[Table 12]**

| Time (min) | 0 | 5 | 7 | 7.1 | 12 |
|---|---|---|---|---|---|
| A buffer (%) | 99 | 1 | **1** | 99 | 99 |
| B buffer (%) | **1** | 99 | 99 | **1** | 1 |

Flow speed: 0.3 mL/min
Inflow: 5 µL
Column: Mightysil RP-18 GP 50-2.0 (5 µm) (KANTO CHEMICAL CO., INC.)

### Multiple Reaction Monitoring Conditions

**[Table 13]**

| Analyte | Precursor ions (*m*/*z*) | Fragment ions (*m*/*z*) | Cone voltage (V) | Collision energy (V) |
|---|---|---|---|---|
| GS-SS-SG | 677.1 | 339.0 | 35 | 25 |

In the system where TME-IAM was made to coexist, approximately 70% GS-SS-SG remained even after the incubation at 37°C in a weakly alkaline solution (pH 8.0) for one hour. This was a higher value than that of the system to which the publicly known iodoacetamide derivative HPE-IAM was added. The results are shown in Fig. 7.

In order to detect the active sulfur molecules with high sensitivity, it is important to form a stable derivative. In order to do so, it is necessary to add a high-concentration alkylating agent to a specimen; and it is known that the addition of the high-concentration alkylating agent promotes the decomposition of the active sulfur molecules (NPL 1). So, processing concentration dependency of the active sulfur molecules stabilization activity of TME-IAM was analyzed by using the active sulfur molecules GS-SS-SG.

The following were mixed: 50 µL of 200 mM phosphate buffer (pH 7.0) (final concentration: 100 mM) and 5-20 µL of a TME-IAM solution (final concentration: 0.25, 0.5, 1, 2, 4 mM), 10 µL of 100 µM GS-SS-SG (final concentration: 10 µM), and 20-35 µL of ultrapure water; and the mixture was incubated at 37°C while shielding light. One hour after the start of the reaction, 10 µL of the reaction solution and 90 µL of 0.1% formic acid were mixed, thereby terminating the reaction. By performing the LC-MS/MS analysis by using unreacted 1 µM GS-SS-SG as standard, a residual amount of GS-SS-SG in the reaction solution was analyzed.

### Equipment

Mass spectrometer: Xevo TQD (Waters)
HPLC: Alliance HPLC e2695 (Waters)

### HPLC Gradient Conditions

A buffer: 0.1% formic acid
B buffer: methanol (for LC/MS)

**[Table 14]**

| Time (min) | 0 | 5 | 7 | 7.1 | 12 |
|---|---|---|---|---|---|
| A buffer (%) | 99 | 1 | **1** | 99 | 99 |
| B buffer (%) | **1** | 99 | 99 | **1** | 1 |

Flow speed: 0.3 mL/min
Inflow: 5 µL
Column: Mightysil RP-18 GP 50-2.0 (5 µm) (KANTO CHEMICAL CO., INC.)

### Multiple Reaction Monitoring Conditions

**[Table 15]**

| Analyte | Precursor ions (*m*/*z*) | Fragment ions (*m*/*z*) | Cone voltage (V) | Collision energy (V) |
|---|---|---|---|---|
| GS-SS-SG | 677.1 | 339.0 | 35 | 25 |

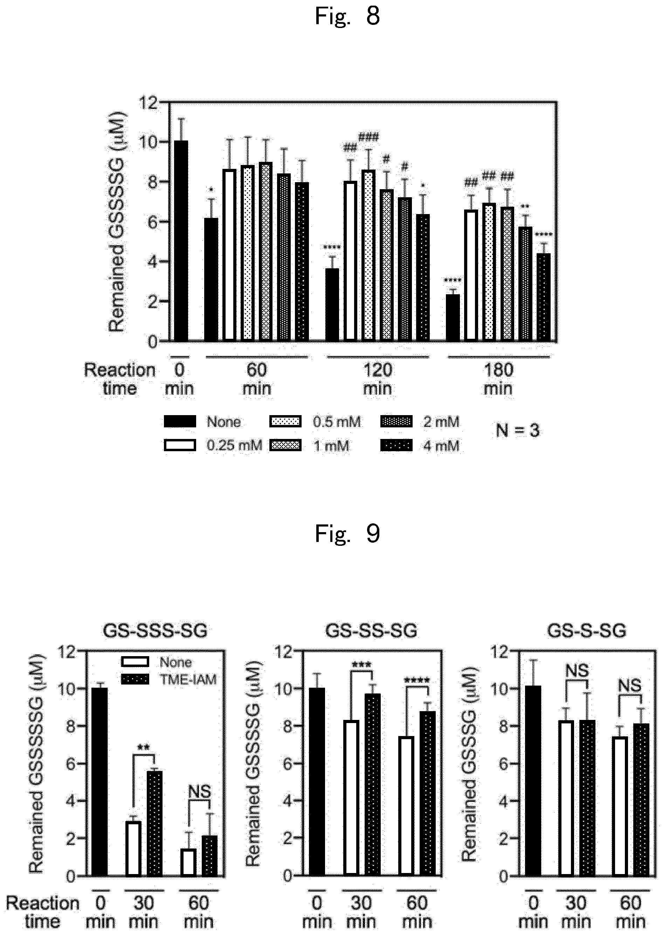

As a result, any significant reduction of GSSSSG was not observed even with a group of GS-SS-SG which was incubated for one hour at 37°C in the presence of 400-fold higher concentration of TME-IAM than GS-SS-SG. In other words, it was found by using TME-IAM that the formation of a derivative of the reactive sulfur molecules and the suppression of the decomposition of its product was very high. The results are shown in Fig. 8.

It has been made clear that compounds with different quantities of sulfur atoms included in the molecules exist in the active sulfur molecules. In general, the compound including a larger number of sulfur atoms has higher reactivity and has unstable molecules. So, the active sulfur molecules stabilization effect of TME-IAM was analyzed by using the active sulfur molecules with different quantities of the contained sulfur atoms. For this analysis, oxidized glutathione pentasulfide (GS-SSS-SG) and oxidized glutathione trisulfide (GS-S-SG) were used in addition to GS-SS-SG.

The following were mixed: 50 µL of 200 mM phosphate buffer (pH 7.0) (final concentration: 100 mM) and 5 µL of 100 mM TME-IAM solution (final concentration: 1 mM), 10 µL of 100 µM oxidized form glutathione polysulfide (GS-SSS-SG, GS-SS-SG, GS-S-SG) (final concentration: 10 µM), and 35 µL of ultrapure water; and the mixture was incubated at 37°C while shielding light. One hour after the start of the reaction, 10 µL of the reaction solution and 90 µL of 0.1% formic acid were mixed, thereby terminating the reaction. By performing the LC-MS/MS analysis by using each unreacted 1 µM oxidized form glutathione polysulfide (GS-SSS-SG, GS-SS-SG, GS-S-SG) as standard, a residual amount of the oxidized form glutathione polysulfide GS-SSS-SG, GS-SS-SG, GS-S-SG) in the reaction solution was analyzed.

### Equipment

Mass spectrometer: Xevo TQD (Waters)
HPLC: Alliance HPLC e2695 (Waters)

### HPLC Gradient Conditions

A buffer: 0.1% formic acid
B buffer: methanol (for LC/MS)

**[Table 16]**

| Time (min) | 0 | 5 | 7 | 7.1 | 12 |
|---|---|---|---|---|---|
| A buffer (%) | 99 | 1 | 1 | 99 | 99 |
| B buffer (%) | 1 | 99 | 99 | 1 | 1 |

Flow speed: 0.3 mL/min
Inflow: 5 µL
Column: Mightysil RP-18 GP 50-2.0 (5 µm) (KANTO CHEMICAL CO., INC.)

### Multiple Reaction Monitoring Conditions

**[Table 17]**

| Analyte | Precursor ions (*m*/*z*) | Fragment ions (*m*/*z*) | Cone voltage (V) | Collision energy (V) |
|---|---|---|---|---|
| GS-SSS-SG | 709.0 | 371.0 | 35 | 49 |
| GS-SS-SG | 677.1 | 339.0 | 35 | 25 |
| GS-S-SG | 645.1 | 387.0 | 35 | 25 |

As a result, even with the very unstable GS-SSS-SG, predominant decomposition suppression by TME-IAM was observed 30 minutes after the processing. The results are shown in Fig. 9.

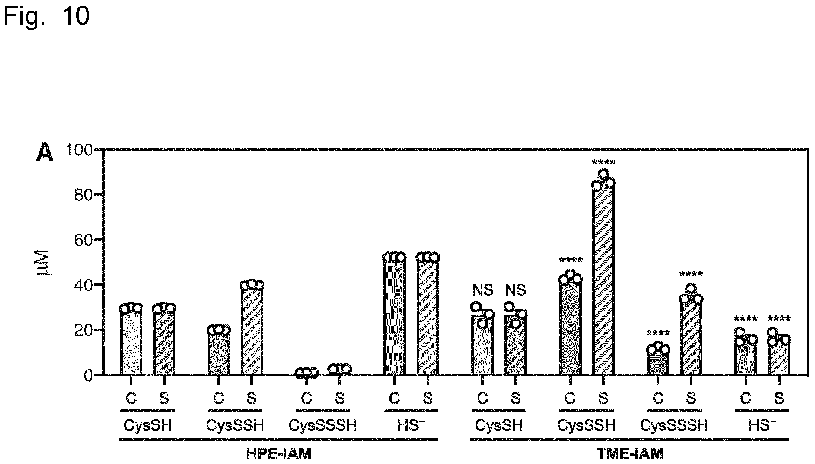

The reactivity of TME-IAM with respect to cysteine hydropolysulfide [CysS-(S)ₙ-H] was verified as compared to HPE-IAM as indicated below. The results are shown in Fig. 10.

CysSSH / CysS-(S)ₙ-H which was prepared by incubating 100 µM cystine and 300 µM Na2S2 for 5 minutes at 37°C was made to react with TME-IAM or HPE-IAM (1 mM each) for one hour at 37°C.

Next, its reaction mixture was provided to the LC-ESI-MS/MS analysis and respective adducts for CysSH / CysS-(S)ₙ-H and HS⁻ (bis-S-adduct) were detected.

Incidentally, the concentration of each compound as sulfur is calculated according to the following formula: a concentration as sulfur = a concentration as the compound × the number of sulfur atoms in the compound (for example, in a case of CysSSH, the concentration as sulfur = 2×[CysSSH]). Referring to Fig. 10, the concentrations of each adduct as the compound and sulfur are indicated as a bar with slanted stripes and a bar without stripes, respectively.

The amounts of CysSSH and cysteine hydrosulfide (CysSSSH) detected by using TME-IAM was significantly larger than those detected by using HPE-IAM as indicated in Fig. 10. On the other hand, regarding the amount of a product deriving from the decomposition of HS⁻ (bis-S adduct) hydrogen sulfide and persulfide / polysulfide, the amount of the product of the HPE-IAM group was prominently higher than the amount of the product detected by using TME-IAM.

This result suggests that while TME-IAM reacts with a thiol residue of hydropolysulfide via an IAM group and forms a stable adduct, its hydroxyphenyl part inhibits hydrolysis and thereby stabilizes polysulfide.

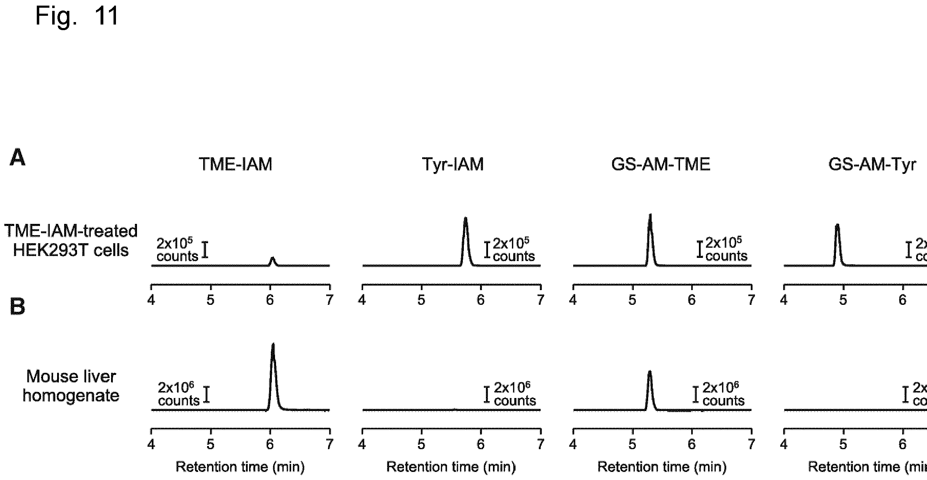

The following test was conducted with regard to cell permeability and esterase sensitivity of TME-IAM. The results are shown in Fig. 11. Fig. 11 illustrates representative mass chromatographs of (A) HEK (human embryonic kidney) 293T cells and (B) mice liver homogenate which were processed with TME-IAM.

After processing the HEK 293T cells for one hour at 37°C by using 1 mM TME-IAM, they were homogenized by ultrasonication in 80% methanol. The mice liver tissues were also homogenized by ultrasonication in 80% methanol containing 1 mM TME-IAM and were incubated for one hour at 37°C. After centrifugal separation of each of them, the obtained supernatant was collected and then diluted to 10 times by using 0.1% FA (formic acid).

Next, the mixture was made to undergo the LC-ESI-MS/MS analysis and individual byproducts driving from TME-IAM, its glutathione adduct (GS-AM-TME), and esterase dependency cleavage were detected. They are Tyr-IAM ((2-iodoacetyl)-I-tyrosine) and GS-AM-Tyr (GSH adduct of (2-iodoacetyl)-1-tyrosine), respectively.

In the MS analysis, both TME-IAM and (2-iodoacetyl)-1-tyrosine (Tyr-IAM) (a product deriving from esterase dependency cleavage of a methyl ester part of TME-IAM in TME-IAM processing) were observed in the human embryonic kidney (HEK) 293T cells. (Fig. 11A)

Furthermore, both the TME-IAM adduct of GSH and its by-product deriving from the esterase dependency cleavage (GSH adduct of (2-iodoacetyl)-1-tyrosine [GS-AM-Tyr]) were observed in the cells which underwent the TME-IAM processing. (Fig. 11A)

These results show that TME-IAM can permeate through cell membranes and react with thiol; however, there is a possibility that the alkylating agent may be rapidly decomposed by a product cleaved at the methyl ester part by intracellular esterase.

Accordingly, in order to evaluate the possibility of forming these derivatives when in vivo samples are used for the reactivity sulfur metabolome analysis, mice liver tissues were prepared in 80% methanol containing 1 mM TME-IAM, homogenized, and incubated for one hour at 37°C, and the obtained samples were analyzed by LC-ESI-MS/MS.

As a result, Tyr-IAM which is an esterase-dependent by-product of TME-IAM was rarely detected in the in vivo samples homogenized in 80% methanol containing TME-IAM. (Fig. 11B)

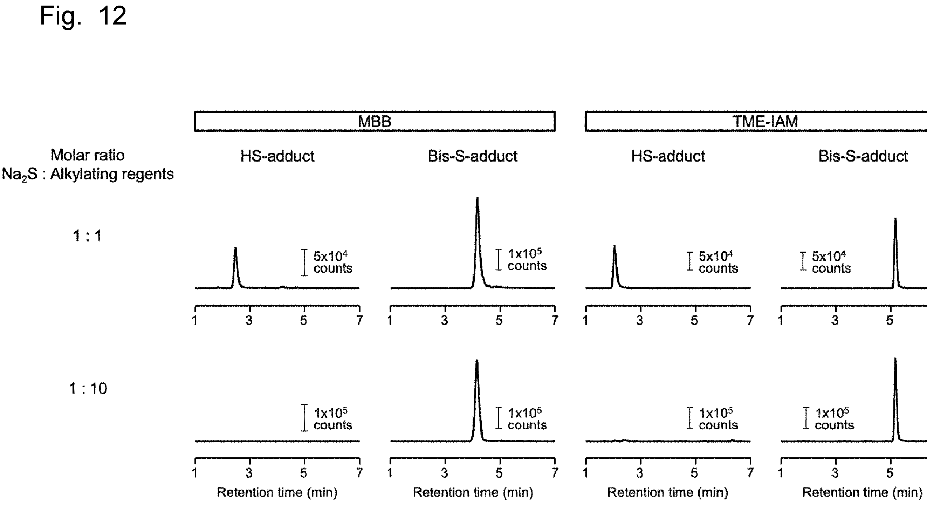

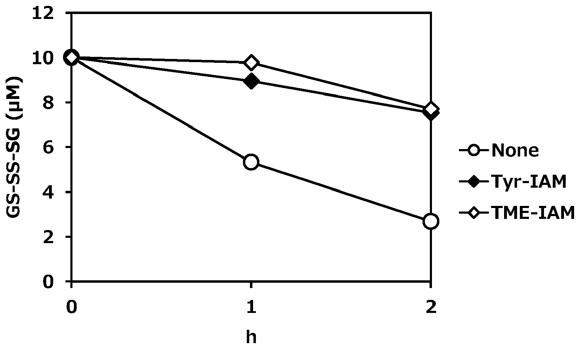

Fig. 12 shows the results of testing the reactivity of TME-IAM with respect to hydrogen sulfide with reference to the reactivity of MBB. Fig. 12 shows representative mass chromatographs when a molar ratio of Na2S to the alkylating agent was 1:1 (upper) or 1:10 (lower).

The incubation of 0.1 mM or 1 mM TME-IAM (right side in Fig. 12) or MBB (left side in Fig. 12) was performed for one hour at 37°C under the existence of 0.1 mM Na2S. As a result, the HS-adduct and the bis-S-adduct in the reaction mixture were respectively detected by the LC-ESI-MS/MS analysis.

The reaction mixture was separated by the Alliance e2695 system equipped with a YMC-Triart C18 column (inside diameter: 50×2.0 mm) and was then eluted with a linear gradient (30-99% for 5 minutes) by using methanol as a mobile phase under the existence of 0.1% FA (formic acid) at a flow speed of 0.3 mL/min at 40°C.

As a result, this experiment successfully confirmed that both MBB and TME-IAM react with hydrogen sulfide and form the bis-S-adduct.

It was also made clear that N-iodoacetyl tyrosine (e in the structural formula of the tyrosine derivative of the present invention is substituted with OH) has the same level of the GS-SS-SG protection effect as that of TME-IAM. The results are shown in Fig. 12. Incidentally, N-iodoacetyl tyrosine is expressed with the following chemical formula.

### N-iodoacetyl tyrosine:

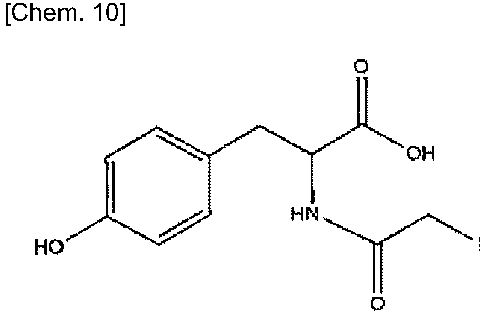

## Claims

1. A new tyrosine derivative formed according to a chemical formula indicated below,
wherein one of a-c is "OH" and the rest are "H";
d is CₙH₂ₙα, where α is "H" or a "halogen atom," the halogen atom is preferably "I," and
n is an integer from 1 to 5; and
e is (NH)ₓ(CO)_{y}C_{z}H_{2z}β, where x is 1 or 0, y is 1 or 0, z is an integer from 1 to 5, β is "H" or a "halogen atom,".

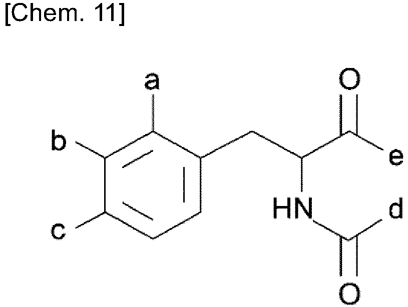

2. The new tyrosine derivative according to claim 1, wherein the new tyrosine derivative is formed according to a chemical formula indicated below.

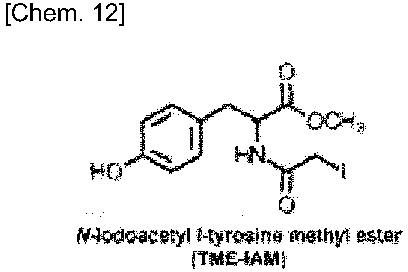

3. The new tyrosine derivative according to claim 1, wherein the new tyrosine derivative is formed according to a chemical formula indicated below.

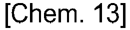

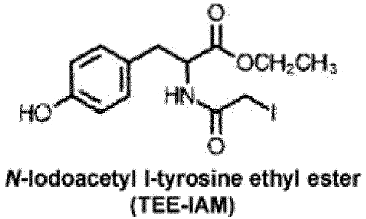

4. The new tyrosine derivative according to claim 1, wherein the new tyrosine derivative is formed according to a chemical formula indicated below.

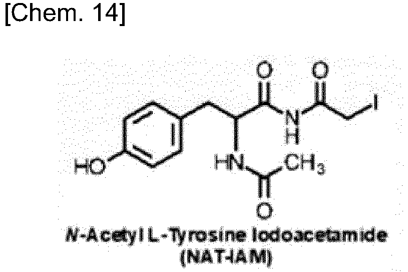

5. A compound for suppressing hydrolysis of polysulfide, the compound containing the tyrosine derivative stated in any one of claims 1 to 4 as its main component.

6. The compound according to claim 5, wherein the tyrosine derivative combines with a thiol group of the polysulfide.

7. The compound according to claim 5, wherein the compound combines with a thiol group of the polysulfide to alkylate the thiol group.

8. A polysulfide analysis method for detecting the compound which is stated in claim 5 and combines with polysulfide, to enable qualitative and quantitative analysis of the polysulfide.

9. A method for suppressing hydrolysis of polysulfide by causing the compound stated in claim 5 to act on the polysulfide.
